(19) Europäisches Patentamt
European Patent Office
Office européen des brevets

(11) Veröffentlichungsnummer: **0 037 019**
**A1**

(12) # EUROPÄISCHE PATENTANMELDUNG

(21) Anmeldenummer: **81102064.3**

(22) Anmeldetag: **19.03.81**

(51) Int. Cl.³: **C 07 C 103/365**
C 07 C 103/375, A 01 N 37/22

(30) Priorität: **01.04.80 DE 3012623**

(43) Veröffentlichungstag der Anmeldung:
**07.10.81 Patentblatt 81/40**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Stetter, Jörg, Dr.**
**Gellertweg 4**
**D-5600 Wuppertal 1(DE)**

(72) Erfinder: **Lürssen, Klaus, Dr.**
**August-Kierspel-Strasse 89**
**D-5070 Bergisch-Gladbach(DE)**

(72) Erfinder: **Eue, Ludwig, Dr.**
**Paul-Klee-Strasse 36**
**D-5090 Leverkusen(DE)**

(72) Erfinder: **Schmidt, Robert-Rudolf, Dr.**
**Hahnenweg 5**
**D-5000 Köln(DE)**

(54) **N-Allenyl-halogenacetanilide, Verfahren zu ihrer Herstellung sowie ihre Verwendung als Wachstumsregulatoren und Herbizide.**

(57) Neue N-Allenyl-halogenacetanilide der allgemeinen Formel

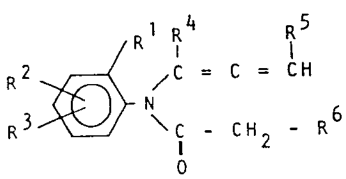
(I)

in welcher
$R^1$ für Wasserstoff, Alkyl oder Halogen steht,
$R^2$ für Wasserstoff oder Alkyl steht,
$R^3$ für Wasserstoff oder Alkyl steht,
$R^4$ für Wasserstoff oder Alkyl steht,
$R^5$ für Wasserstoff oder Alkyl steht und
$R^6$ für Halogen steht,
ihre Herstellung durch Umlagerung von N-Propargyl-Halogenacetaniliden sowie ihre Verwendung als Wachstumsregulatoren und Herbizide.

EP 0 037 019 A1

BAYER AKTIENGESELLSCHAFT 5090 Leverkusen, Bayerwerk

Zentralbereich
Patente, Marken und Lizenzen Dü/Th
I a

N-Allenyl-halogenacetanilide, Verfahren zu ihrer Herstellung sowie ihre Verwendung als Wachstumsregulatoren und Herbizide

Die vorliegende Erfindung betrifft neue N-Allenyl-halogenacetanilide, ein Verfahren zu iher Herstellung sowie ihre Verwendung als Wachstumsregulatoren und Herbizide.

Es ist bereits bekannt geworden, daß (2-Chlorethyl)-trimethyl-ammoniumchlorid, Maleinsäurehydrazid und Bernsteinsäure-mono-N-dimethyl-hydrazid pflanzenwachstumsregulierende Eigenschaften aufweisen (vgl. R. Wegler, Chemie der Pflanzenschutz- und Schädlingsbekämpfungsmittel, Band 5, Seiten 167, 271 und 315, Springer Verlag (1977)). Die Wirksamkeit dieser Stoffe ist jedoch vor allem bei niedrigen Aufwandmengen, nicht immer voll befriedigend.

Es wurden neue N-Allenyl-halogenacetanilide der allgemeinen Formel

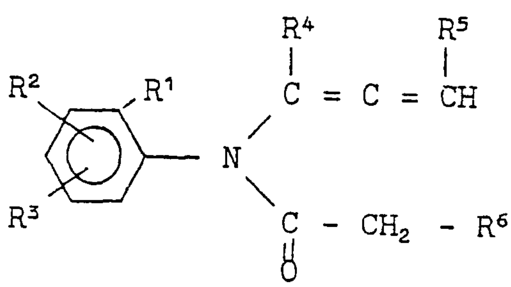

(I)

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff oder Alkyl steht,

$R^5$ für Wasserstoff oder Alkyl steht und

$R^6$ für Halogen steht,

gefunden.

Weiterhin wurde gefunden, daß man die neuen N-Allenyl-halogenacetanilide der Formel (I) erhält, wenn man N-Propargyl-halogenacetanilide der Formel

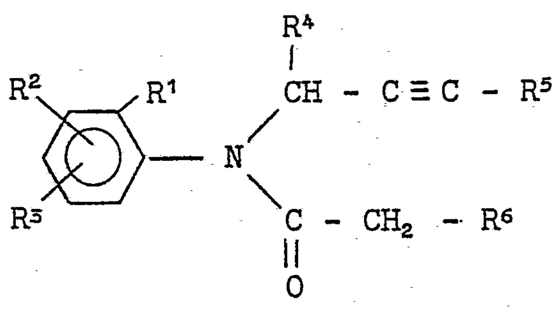

(II)

in welcher

$R^1$, $R^2$, $R^3$, $R^4$, $R^5$ und $R^6$ die oben angegebene Bedeutung haben,

in Gegenwart einer Base als Katalysator und gegebenenfalls in Gegenwart eines Verdünnungsmittels umlagert.

Weiterhin wurde gefunden, daß die neuen N-Allenyl-halogenacetanilide der Formel (I) starke pflanzenwachstumsregulierende und herbizide Eigenschaften aufweisen.

Überraschenderweise zeigen die erfindungsgemäßen Stoffe der Formel (I) eine bessere pflanzenwachstumsregulierende Wirkung als die bekannten Verbindungen (2-Chlorethyl)-trimethylammoniumchlorid, Maleinsäurehydrazid und Bernsteinsäure-mono-N-dimethyl-hydrazid, welches anerkannt gut wirksame Stoffe gleicher Wirkungsart sind. Die erfindungsgemäßen Verbindungen stellen somit eine Bereicherung der Technik dar.

Die erfindungsgemäßen N-Allenyl-halogenacetanilide sind durch die Formel (I) allgemein definiert. In dieser Formel stehen $\underline{R}^1$, $\underline{R}^2$, $\underline{R}^3$, $\underline{R}^4$ und $\underline{R}^5$ jeweils vorzugsweise für Wasserstoff und geradkettiges oder verzweigtes Alkyl mit 1 bis 4 Kohlenstoffatomen, $\underline{R}^1$ steht außerdem vorzugsweise für Halogen, wie insbesondere Fluor, Chlor oder Brom. $\underline{R}^6$ steht vorzugsweise für Chlor, Brom und Jod.

Ganz besonders bevorzugt sind diejenigen N-Allenyl-halogenacetanilide der Formel (I), in denen $\underline{R}^1$, $\underline{R}^2$ und $\underline{R}^3$ für Wasserstoff, Methyl, Ethyl, Isopropyl, sek.-Butyl und tert.-Butyl stehen; $\underline{R}^1$ außerdem für Chlor oder Brom steht; $\underline{R}^4$ und $\underline{R}^5$ für Wasserstoff, Methyl oder Ethyl stehen; und $\underline{R}^6$ für Chlor oder Brom steht.

Im einzelnen seien außer den bei den Herstellungsbeispielen genannten Verbindungen die folgenden Verbindungen der allgemeinen Formel (I) genannt:

Tabelle 1:

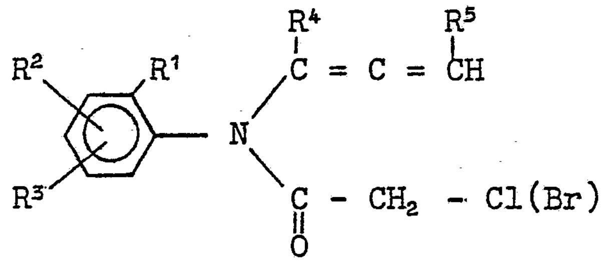

(Ia)

| $R^1$ | $R^2$ | $R^3$ | $R^4$ | $R^5$ |
|---|---|---|---|---|
| Cl | 6-$CH_3$ | H | H | H |
| $C(CH_3)_3$ | H | H | H | H |
| $CH_3$ | 3-$CH_3$ | 6-$CH_3$ | H | H |
| H | H | H | H | H |
| $CH_3$ | 3-$CH_3$ | H | H | H |
| $CH_3$ | 6-$CH_3$ | H | $CH_3$ | H |
| $CH_3$ | 6-$C_2H_5$ | H | $CH_3$ | H |
| $C_2H_5$ | 6-$C_2H_5$ | H | $CH_3$ | H |
| Cl | 6-$CH_3$ | H | $CH_3$ | H |
| $C(CH_3)_3$ | H | H | $CH_3$ | H |
| $CH_3$ | 3-$CH_3$ | 6-$CH_3$ | $CH_3$ | H |
| H | H | H | $CH_3$ | H |
| $CH_3$ | 3-$CH_3$ | H | $CH_3$ | H |
| $CH_3$ | 6-$CH_3$ | H | H | $CH_3$ |
| $CH_3$ | 6-$C_2H_5$ | H | H | $CH_3$ |
| $C_2H_5$ | 6-$C_2H_5$ | H | H | $CH_3$ |
| Cl | 6-$CH_3$ | H | H | $CH_3$ |
| $C(CH_3)_3$ | H | H | H | $CH_3$ |
| $CH_3$ | 3-$CH_3$ | 6-$CH_3$ | H | $CH_3$ |
| H | H | H | H | $CH_3$ |
| $CH_3$ | 3-$CH_3$ | H | H | $CH_3$ |

Tabelle 1 (Fortsetzung)

| $R^1$ | $R^2$ | $R^3$ | $R^4$ | $R^5$ |
|---|---|---|---|---|
| $CH_3$ | $6\text{-}CH_3$ | H | $CH_3$ | $CH_3$ |
| $CH_3$ | $6\text{-}C_2H_5$ | H | $CH_3$ | $CH_3$ |
| $C_2H_5$ | $6\text{-}C_2H_5$ | H | $CH_3$ | $CH_3$ |
| Cl | $6\text{-}CH_3$ | H | $CH_3$ | $CH_3$ |
| $C(CH_3)_3$ | H | H | $CH_3$ | $CH_3$ |
| $CH_3$ | $3\text{-}CH_3$ | $6\text{-}CH_3$ | $CH_3$ | $CH_3$ |
| H | H | H | $CH_3$ | $CH_3$ |
| $CH_3$ | $3\text{-}CH_3$ | H | $CH_3$ | $CH_3$ |

Verwendet man beispielsweise 2,6-Dimethyl-N-propargyl-chloracetanilid als Ausgangsstoffe und Kalium-tert.-butylat als Base, so kann der Ablauf des erfindungsgemäßen Verfahrens durch das folgende Formelchema wiedergegeben werden:

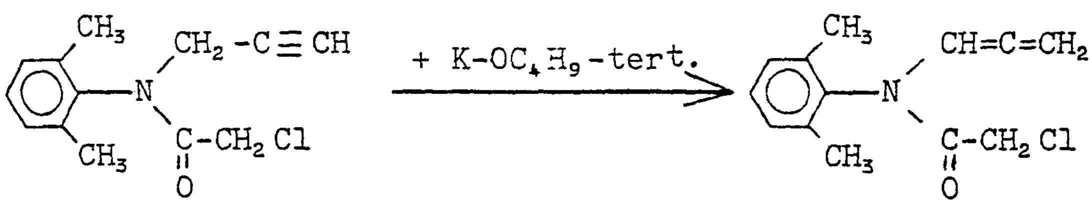


Die bei der Durchführung des erfindungsgemäßen Verfahrens als Ausgangsstoffe benötigten N-Propargyl-halogen-acetanilide sind durch die Formel (II) allgemein definiert. In dieser Formel stehen $R^1$, $R^2$, $R^3$, $R^4$, $R^5$ und $R^6$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-Propargyl-halogenacetanilide der Formel (II) sind teilweise bekannt (vergleiche DE-OS 1 542 702), teilweise sind sie Gegenstand einer eigenen älteren Anmeldung (vergleiche DE-OS 29 19 196).

Sie können nach den dort beschriebenen Verfahren erhalten werden, indem man

a) Propargyl-aniline der Formel

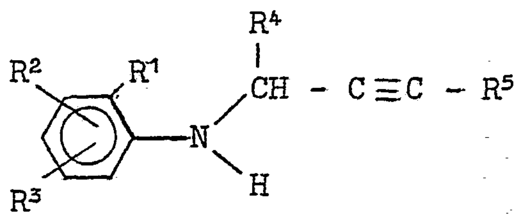

(III)

in welcher

$R^1$, $R^2$, $R^3$, $R^4$ und $R^5$ die oben angegebene Bedeutung haben,

mit Halogenessigsäurechloriden oder -bromiden bzw.-anhydriden der Formeln

$R^6 -CH_2 - CO - Cl(Br)$ (IVa)

bzw.

$(R^6 - CH_2 - CO)_2O$ (IVb)

in welcher

$R^6$ die oben angegebene Bedeutung hat,

in Gegenwart eines inerten organischen Lösungsmittels, wie beispielsweise Tetrahydrofuran, und gegebenenfalls in Gegenwart eines Säurebindemittels, wie beispielsweise Triethylamin oder Pyridin, bei Temperaturen zwischen 20 und 100°C umsetzt, oder

b) Halogenacetanilide der Formel

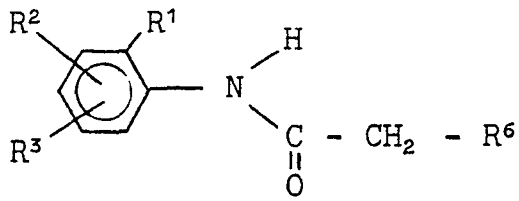

(V)

in welcher

$R^1$, $R^2$, $R^3$ und $R^6$ die oben angegebene Bedeutung haben,

mit Propargylhalogeniden der Formel

$$Hal-\overset{\overset{R^4}{|}}{C}H-C\equiv C-R^5 \qquad (VI)$$

in welcher

$R^4$ und $R^5$ die oben angegebene Bedeutung haben und

Hal für Chlor oder Brom steht,

vorzugsweise in einem wässrig-organischen Zweiphasensystem, wie beispielsweise wässrige Natron- oder Kalilauge/Toluol oder Methylenchlorid, gegebenenfalls unter Zusatz von 0,1 - 1 Mol eines Phasen-Transferkatalysators, wie beispielsweise Triethyl-benzyl-ammonium-chlorid, bei Temperaturen zwischen -20 und + 80°C umsetzt.

Die N-Propargylaniline der Formel (III) sind teilweise bekannt (vergleiche die US-Patentschriften 3 535 377 und 4 001 325), bzw. können sie nach bekannten Verfahren erhalten werden, indem man z.B. entsprechende Aniline mit Propargylhalogeniden der Formel (VI) oder den entsprechenden Propargylsulfonaten, wie beispielsweise Mesylaten oder Tosylaten, in Gegenwart eines Säurebinders, wie z.B.

Natrium- oder Kaliumcarbonat, gegebenenfalls in Gegenwart eines inerten organischen Lösungsmittel, wie beispielsweise Ethanol, bei Temperaturen zwischen 20°C und 150°C umsetzt, wobei vorzugsweise ein Überschuß an Anilin eingesetzt werden kann.

N-Propargyl-aniline der Formel (III), bei denen $R^4$ für Methyl steht, können auch durch Umsetzung der entsprechenden Aniline mit Acetylen in Gegenwart von Kupferacetylid unter Druck erhalten werden (vergleiche hierzu Liebigs Ann. Chem. 596, 1 (1955)).

Die Halogenessigsäurechloride und -bromide bzw. -anhydride der Formeln (IVa) und (IVb) sowie die Propargylhalogenide der Formel (VI) sind allgemein bekannte Verbindungen der organischen Chemie.

Die Halogenacetanilide der Formel (V) sind allgemein bekannt, bzw. können sie in allgemein bekannter Art und Weise erhalten werden, indem man entsprechende Aniline mit einem Halogenessigsäurechlorid oder -bromid bzw. -anhydrid der Formeln (IVa) und (IVb) in Gegenwart eines inerten organischen Lösungsmittels, wie beispielsweise Toluol oder Dimethylformamid, gegebenenfalls in Gegenwart eines Säurebindemittels, wie z.B. Kaliumcarbonat oder Triethylamin, bei Temperaturen zwischen 0 und 100°C umsetzt.

Als Verdünnungsmittel kommen für die erfindungsgemäße Umsetzung vorzugsweise inerte organische Lösungsmittel infrage. Hierzu gehören vorzugsweise Ether, wie Tetrahydrofuran oder Dioxan; Alkohole, wie Methanol, Ethanol oder tert.-Butanol; sowie Dimethylsulfoxid.

Das erfindungsgemäße Verfahren wird in Gegenwart einer Base als Katalysator durchgeführt. Hierzu werden vorzugsweise Alkalihydroxide oder Alkalicarbonat, wie beispielsweise Natrium- oder Kalium-hydroxid bzw. -carbonat, und insbesondere Alkalialkoholate, wie beispielsweise Natriummethylat, Natriumethylat oder Kalium-tert.-butylat verwendet.

Die Reaktionstemperaturen können bei der Durchführung des erfindungsgemäßen Verfahrens in einem größeren Bereich variiert werden. Im allgemeinen arbeitet man zwischen 0 und 120°C, vorzugsweise zwischen 20 und 80°C.

Bei der Durchführung des erfindungsgemäßen Verfahrens behandelt man N-Propargyl-halogenacetanilide der Formel (II) gegebenenfalls in Gegenwart eines Verdünnungsmittels mit einer katalytischen Menge an Base. Die Aufarbeitung erfolgt nach üblichen Methoden. Im allgemeinen verfährt man in der Weise, daß man das Reaktionsgemisch nach beendeter Umsetzung in Wasser gibt, das dabei anfallende Festprodukt abfiltriert, trocknet und gegebenenfalls umkristallisiert.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten klei-

neren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak,

Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im wesentlichen Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattung: Sinapsis, Lepidium, Galium, Stellaria, Matricaria, Anthemis, Galinsoga, Chenopodium, Urtica, Senecio, Amaranthus, Portulaca, Xanthium, Convolvulus, Ipomcea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattung: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattung: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattung: Oryzae, Zea, Triticum, Hordeum, Avena, Secala, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölze-, Obst-, Wein-, Citrus-, Nuss-, Bananen-, Tee-, Oelpalm-, Kakao-, Beerenfrucht- und Hopfenanlagen und zur selektiven Unkrautbekämpfung in einjährigen Kulturen eingesetzt werden.

Besonders geeignet sind die erfindungsgemäßen Wirkstoffe zum selektiven Einsatz in Zuckerrüben, Sojabohnen und anderen Leguminosen sowie in Mais und Baumwolle.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch vermischen der Wirkstoffe mit Streckmitteln, also flüssigen Lösungsmitteln, unter Druck stehenden verflüssigten Gasen und / oder festen Trägerstoffen, gegebenenfalls unter Verwendung von oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie

Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen o,1 und 95 Gewichtsprozent Wirkstoff, vorzugsweise zwischen o,5 und 9o %.

Die erfindungsgemäßen Wirkstoffe können als solche oder in ihren Formulierungen auch in Mischung mit bekannten Herbiziden oder Pflanzenwucherregulatoren Verwendung finden, wobei Fertigformulierungen oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angewendet werden. Die Anwendung geschieht in üblicher Weise, z.B. durch Gießen,

Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei einer Anwendung der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Bei einer Anwendung der erfindungsgemäßen Wirkstoffe als Herbizide können die Wirkstoffe sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Die Wirkstoffe können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,01 und 50 kg Wirkstoff pro ha. vorzugsweise zwischen 0,05 und 10 kg/ha.

Die Herstellung und die Verwendung der erfindungsgemäßen Stoffe geht aus den nachfolgenden Beispielen hervor.

Herstellungsbeispiele

Beispiel 1

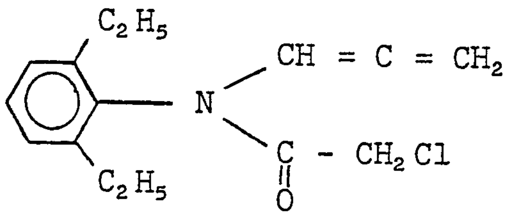

30 g (0,11 Mol) 2,6-Diethyl-N-propargyl-chloracetanilid werden in 400 ml tert.-Butanol gelöst, und diese Lösung wird anschließend mit 200 mg Kalium-tert.-butylat versetzt. Nach 1 Stunde Rühren bei 20°C ist die Umlagerung vollständig (dünnschichtchromatographisch nachgewiesen). Das Reaktionsgemisch wird auf Wasser gegeben, das ausfallende kristalline Reaktionsprodukt abgesaugt, getrocknet und aus Petrolether umkristallisiert. Man erhält 19,1g (64 % der Theorie) 2,6-Diethyl-N-allenyl-chloracetanilid vom Schmelzpunkt 66°C (Das $^1$H-NMR-Spektrum zeigt die vollständige Isomerisierung an).

In analoger Weise werden die folgenden Verbindungen der allgemeinen Formel

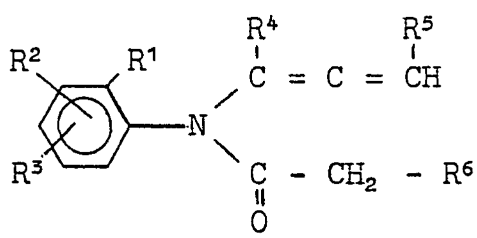

(I)

erhalten:

Tabelle 2

| Bsp. Nr. | $R^1$ | $R^2$ | $R^3$ | $R^4$ | $R^5$ | $R^6$ | Schmelzpunkt(°C) |
|---|---|---|---|---|---|---|---|
| 2 | $CH_3$ | 6-$CH_3$ | H | H | H | Cl | 112-15 |
| 3 | $CH_3$ | 6-$C_2H_5$ | H | H | H | Cl | 58-59 |
| 4 | $C(CH_3)_3$ | H | H | H | H | Cl | 107-109 |
| 5 | Cl | 6-$CH_3$ | H | H | H | Cl | 117-119 |

Anwendungsbeispiele

In diesen Beispielen werden die nachstehend angegebenen Verbindungen als Vergleichssubstanzen eingesetzt.

A = $[Cl-CH_2-CH_2-N(CH_3)_3]^{\oplus}\ Cl^{\ominus}$

(2-Chlorethyl)-trimethyl-ammoniumchlorid

B =

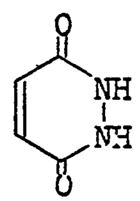

Maleinsäurehydrazid

C = $(CH_3)_2N-NH-C(=O)-CH_2-CH_2-C(=O)-OH$

Bernsteinsäure-mono-N-dimethyl-hydrazid

Beispiel A



Wuchshemmung bei Gerste

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstenpflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend der Kontrollpflanzen.

Die erfindungsgemäßen Wirkstoffe 1 und 3 zeigen in diesem Test eine bessere Wuchshemmung als die aus dem Stand der Technik bekannte Substanz (A).

Beispiel B

Wuchshemmung bei Gras (Festuca pratensis)

Lösungsmittel: 30 Gewichtsteile Dimethylformamid

Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnass besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstumes und 0 % ein Wachstum entsprechend dem der Kontrollpflanzen.

Die erfindungsgemäßen Wirkstoffe 1 und 2 zeigen in diesem Test eine bessere Wuchshemmung als die aus dem Stand der Technik bekannte Verbindung (B).

Beispiel C

Wuchsbeeinflussung bei Zuckerrüben

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Die erfindungsgemäßen Wirkstoffe 1 und 2 zeigen in diesem Test eine bessere Wuchbeeinflussung als die aus dem Stand der Technik bekannte Verbindung (C).

Beispiel D

Pre-emergence-Test

Lösungsmittel: 5 Gewichtsteile Aceton

Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 %=keine Wirkung (wie unbehandelte Kontrolle)

100 %=totale Vernichtung

Die erfindungsgemäßen Wirkstoffe 1, 2 und 3 zeigen in diesem Test eine gute herbizide Wirksamkeit, insbesondere eine selektiv herbizide Wirkung.

Patentansprüche

1) N-Allenyl-halogenacetanilide der Formel

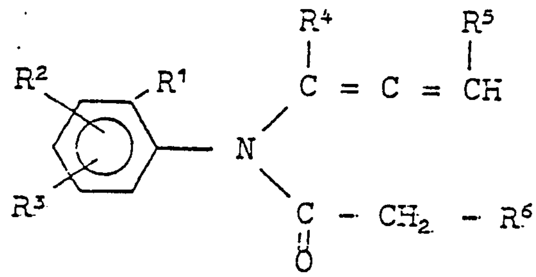

(I)

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff oder Alkyl steht,

$R^5$ für Wasserstoff oder Alkyl steht und

$R^6$ für Halogen steht.

2. N-Allenyl-halogenacetanilide der Formel (I) gemäß Anspruch 1, in denen $R^1$ für Wasserstoff, Alkyl mit 1 bis 4 Kohlenstoffatomen, Fluor, Chlor

oder Brom steht, $R^2$ für Wasserstoff oder Alkyl mit 1 bis 4 Kohlenstoffatomen steht, $R^3$ für Wasserstoff oder Alkyl mit 1 bis 4 Kohlenstoffatomen steht, $R^4$ für Wasserstoff oder Alkyl mit 1 bis 4 Kohlenstoffatomen sthet, $R^5$ für Wasserstoff oder Alkyl mit 1 bis 4 Kohlenstoffatomen steht und $R^6$ für Chlor, Brom oder Jod steht.

3) N-Allenyl-halogenacetanilid der Formel

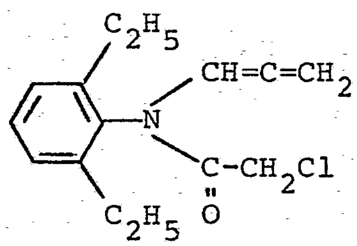

4) N-Allenyl-halogenacetanilid der Formel

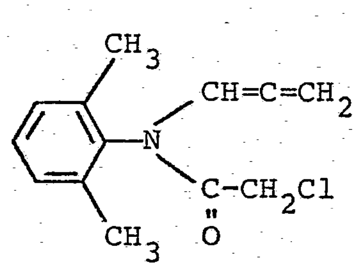

5) N-Allenyl-halogenacetanilid der Formel

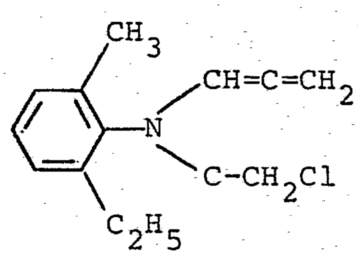

6) Verfahren zur Herstellung von N-Allenyl-halogenacet=aniliden der Formel

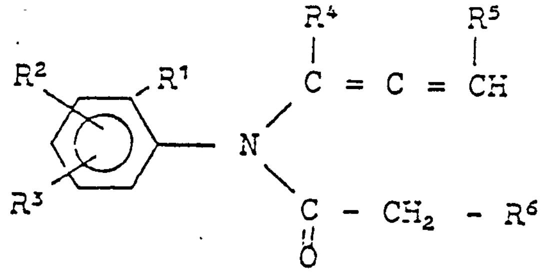

(I)

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff oder Alkyl steht,

$R^5$ für Wasserstoff oder Alkyl steht und

$R^6$ für Halogen steht,

dadurch gekennzeichnet,
daß man N-Propargyl-halogenacetanilide der Formel

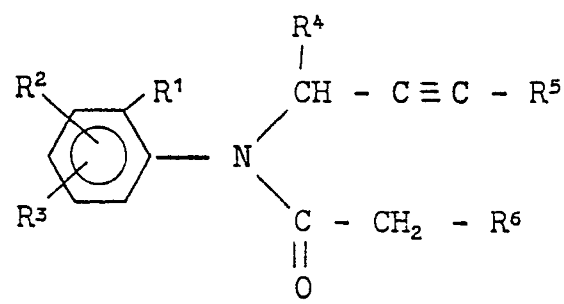

(II)

in welcher

$R^1$, $R^2$, $R^3$, $R^4$, $R^5$ und $R^6$ die oben angegebene Bedeutung haben,

in Gegenwart einer Base als Katalysator und gegebenenfalls in Gegenwart eines Verdünnungsmittels umlagert.

7) Herbizide und pflanzenwuchsregulierende Mittel, gekennzeichnet durch einen Gehalt an mindestens einem N-Allenyl-halogenacetanilid der Formel (I).

8) Verfahren zur Bekämpfung von Unkräutern sowie zur Regulierung des Pflanzenwachstums, dadurch gekennzeichnet, daß man N-Allenyl-halogenacetanilide der Formel (I) auf Unkräuter bzw. die zu regulierenden Pflanzen und/oder deren Lebensraum ausbringt.

9) Verwendung von N-Allenyl-halogenacetaniliden der Formel (I) zur Bekämpfung von Unkräutern sowie zur Regulierung des Pflanzenwachstums.

10) Verfahren zur Herstellung von herbiziden bzw. pflanzenwuchsregulierenden Mitteln, dadurch gekennzeichnet, daß man N-Allenyl-halogenacetanilide der Formel (I) mit Streckmitteln und/oder oberflächenaktiven Stoffen vermischt.

0037019

Europäisches Patentamt

# EUROPÄISCHER RECHERCHENBERICHT

Nummer der Anmeldung
EP 81 10 2064

| Kategorie | EINSCHLÄGIGE DOKUMENTE — Kennzeichnung des Dokuments mit Angabe, soweit erforderlich, der maßgeblichen Teile | betrifft Anspruch | KLASSIFIKATION DER ANMELDUNG (Int Cl.) |
|---|---|---|---|
| A | TETRAHEDRON LETTERS, Nr. 7, Februar 1979, Seiten 599-600 Oxford, G.B. L.E. OVERMAN et al.: "The preparation of n-trichloroacetamido-1, 2-dienes" * Insgesamt * -- | 1,6 | C 07 C 103/365 103/375 A 01 N 37/22 |
| DA | DE - A - 2 919 196 (BAYER A.G.) * Ansprüche * -- | 1,7-10 | |
| | | | RECHERCHIERTE SACHGEBIETE (Int Cl.) |
| A | DE - A - 2 362 743 (DIAMOND SHAMROCK CORP.) * Ansprüche * -- | 1,7-10 | C 07 C 103/365 103/375 |
| DA | DE - A - 1 542 702 (B.A.S.F.) * Seiten 1-5 * -- | 1,7-10 | |
| P | EP - A - 0 018 509 (BAYER A.G.) * Anspruch 6 * ---- | 1,6-10 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 26-06-1981 | PAUWELS |

EPA form 1503.1 06.78